# EUROPEAN PATENT SPECIFICATION

(11) **EP 2 423 302 B1**
(45) Date of publication and mention of the grant of the patent: **08.07.2015**
(21) Application number: 09843569.6
(22) Date of filing: 30.09.2009
(51) Int. Cl.: C12N 5/071, C12N 5/10, C12Q 1/68

(54) **NEW SERUM-FREE MEDIUM FOR INDUCING PLURIPOTENT STEM CELLS QUICKLY WITH HIGH EFFICIENCY AND METHOD USING THEREOF**
NEUES SERUMFREIES MEDIUM ZUR HOCHEFFIZIENTEN SCHNELLEN INDUZIERUNG PLURIPOTENTER STAMMZELLEN UND VERWENDUNGSVERFAHREN DAFÜR
NOUVEAU MILIEU SANS SÉRUM POUR L'INDUCTION RAPIDE DE CELLULES SOUCHES PLURIPOTENTES AVEC UN RENDEMENT ÉLEVÉ ET SON PROCÉDÉ D'UTILISATION

(30) Priority: 23.04.2009 CN 200910038883
(43) Date of publication of application: 29.02.2012
(73) Proprietor: Guangzhou Institute Of Biomedicine And Health, Chines Academy Of Sciences, Guangdong 510663 (CN)
(72) Inventor: PEI, Duanqing, Guangzhou Guangdong 510663 (CN); CHEN, Jiekai, Guangzhou Guangdong 510663 (CN); LIU, Jing, Guangzhou Guangdong 510663 (CN)
(74) Representative: Gulde & Partner
(86) International application number: PCT/CN2009/074358
(87) International publication number: WO 2010/121465

(56) References cited:
- WO-A1-2009/023161
- WO-A2-2006/044204
- CN-A- 1 431 299
- CN-A- 1 673 356
- CN-A- 1 775 945
- CN-A- 1 800 368
- CN-A- 1 834 234
- CN-A- 1 844 374
- CN-A- 1 961 068
- CN-A- 1 974 764
- CN-A- 101 072 868
- CN-A- 101 092 606
- CN-A- 101 126 077
- CN-A- 101 333 513
- US-A1- 2009 081 784
- AMIT M ET AL: "Feeder layer- and serum-free culture of human embryonic stem cells", BIOLOGY OF REPRODUCTION, NEW YORK, NY [U.A.] : ACADEM. PRESS, US, vol. 70, 1 January 2004 (2004-01-01), pages 837-845, XP002978624, ISSN: 0006-3363, DOI: 10.1095/BIOLREPROD.103.021147
- TROND AASEN ET AL: "Efficient and rapid generation of induced pluripotent stem cells from human keratinocytes", NATURE BIOTECHNOLOGY, vol. 26, no. 11, 1 November 2008 (2008-11-01), pages 1276-1284, XP055019438, ISSN: 1087-0156, DOI: 10.1038/nbt.1503
- LUDWIG TENNEILLE E ET AL: "Feeder-independent culture of human embryonic stem cells", NATURE METHODS, NATURE PUBLISHING GROUP, GB, vol. 3, no. 8, 1 August 2006 (2006-08-01), pages 637-646, XP008084296, ISSN: 1548-7091, DOI: 10.1038/NMETH902
- VANESSA DING ET AL: "Deciphering the Importance of Three Key Media Components in Human Embryonic Stem Cell Cultures", BIOTECHNOLOGY LETTERS, SPRINGER NETHERLANDS, DORDRECHT, vol. 28, no. 7, 1 April 2006 (2006-04-01), pages 491-495, XP019231244, ISSN: 1573-6776, DOI: 10.1007/S10529-006-0005-8
- CHEN JIEKAI ET AL: "Rational optimization of reprogramming culture conditions for the generation of induced pluripotent stem cells with ultra-high efficiency and fast kinetics", CELL RESEARCH, vol. 21, no. 6, June 2011 (2011-06), pages 884-894, XP002695107, ISSN: 1001-0602

### SEQUENCE LISTING

<110> GUANGZHOU INSTITUTE OF BIOMEDICINE AND HEALTH; CHINESE ACADEMY OF SCIENCES
<120> NEW SERUM-FREE MEDIUM FOR INDUCING PLURIPOTENT STEM CELLS QUICKLY WITH HIGH EFFICIENCY AND METHOD USING THEREOF
<130> P09245WO/EP
<150> 200910038883.4
   <151> 2009-04-23
<150> PCT/CN2009/074358
   <151> 2009-09-30
<160> 2
<170> PatentIn version 3.3
<210> 1
   <211> 25
   <212> DNA
   <213> artificial sequence
<220>
   <223> Primer
<400> 1
   aatgtttatg gtggattttg taggt 25
<210> 2
   <211> 25
   <212> DNA
   <213> artificial sequence
<220>
   <223> Primer
<400> 2
   cccacactca tatcaatata ataac 25

## Description

### FIELD OF THE INVENTION

The present invention relates to a serum-free medium for inducing and reprogramming somatic cells into induced pluripotent stem cells (iPS) quickly with high efficiency, and the method using thereof for inducing and reprogramming somatic cells without feeder, wherein the rate and efficiency of whole process of inducing and reprogramming are greatly improved. Furthermore, the present invention also relates to the uses of the medium in inducing pluripotent stem cells, and the methods for screening compounds, especially high throughput screening compounds .

### BACKGROUND OF THE INVENTION

Stem cells are the initial source of human body and the various histiocytes thereof, and biologically, are prominently characterized by their ability to self-renew and continuously proliferate as well as the potential of multi-directional differentiation. Stem cells are divided into somatic stem cells and embryonic stem cells (ES cells) depending on their sources. The somatic stem cells include bone marrow mesenchymal stem cells, pancreatic stem cells, neural stem cells, or the like in adult tissues.

In 1981, ES cells were successfully isolated and cultured in mice for the first time and mouse ES cells are the most widely studied and most mature stem cell system by far. Soon afterwards, ES cells have been successfully isolated and cultured in succession in large animals such as cattle and sheep.

The studies of hES cells are promising mainly in therapeutic transplantation. In the field of tissue engineering, hES cells are used as seed cells to provide a great amount of materials for clinical transplantation of cells, tissues, or organs. Specific types of histiocytes can be obtained through in vitro induction and differentiation strategies including controlling the differentiation and culture environments of hES cells and transfecting key molecular genes that can promote the directional differentiation of ES cells. Such cells can be used in transplantation, which will bring new hope for treatment of diabetes, Parkinson's diseases, spinal cord injury, leukemia, myocardial damage, renal failure, liver cirrhosis, or other diseases.

The studies of hES have always been confronted with many problems and disputes which mainly include: (1) It is difficult to obtain the source of donor oocytes and the efficiency of establishing hES cell line is low. Furthermore, the SCNT technology is immature, which will inevitably result in higher consumption of human oocytes, so it is difficult to guarantee the source of oocytes. (2) Immunologic rejection may occur. Patients still immunologically reject various cells and tissues differentiated from hES cells unless the SCNT technology is applied. (3) hES cells are tumorigenic and may possibly develop tumors after they are transplanted into the body of a recipient. This problem may not be well solved in spite of the use of the SCNT technology, provision of suicide genes in transplanted cells, or other measures (Reubinoff BE et al., Embryonic stem cell lines from human blastocysts: somatic differentiation in vitro. Nat Biotechnol 2000; 18: 399-404; Richards M et al., Bongso A. Human feeders support prolonged undifferentiated growth of human inner cell masses and embryonic stem cells. Nat Biotechnol 2002; 20:933-936; Burdon T et al., cell cycle and pluripotency in embryonic stem cells. Trends Cell Biol 2002; 12: 432-438). (4) There are risks of maintaining hES in vitro (Nakagawa M et al., N, Yamanaka S. Generation of induced pluripotent stem cells without Myc from mouse and human fibroblasts. Nat Biotechnol 2008; 26: 101-106). Also, the lentivirus transfection technology may suffer similar risks.

To circumvent ethical controversies on hES cells and therapeutic cloning studies, it is necessary to find out an alternative way to directly transform human somatic cells into induced pluripotent stem cells and provide patients with "individualized" autologous stem cells. In 2003, the Gurdon study group found that, after the nuclei of fully differentiated mouse thymocytes or adult peripheral blood lymphocytes were injected into the oocytes of Xenopus laevis, the differentiation marker of mammalian nucleus was lost, while Oct4, the most characteristic marker in mammalian stem cells was highly expressed, indicating that the nuclei of mammalian cells can be directly reconstructed and hence expressed by the nuclear vacuoles of amphibian oocytes (Byrne JA et al., Nuclei of adult mammalian somatic cells are directly reprogrammed to oct-4 stem cell gene expression by amphibian oocytes. Curr Biol 2003; 13: 1206-1213).

In 2006, the Yamanaka study group in Kyoto University (Japan) screened four transcription factors including Oct4, Sox2, c-Myc, and Klf4 from 24 factors using the in vitro gene transfection technology, introduced the four transcription factors above into mouse embryonic fibroblasts or fibroblasts from the tail-tip skin of adult mice through retroviruses, and obtained a Fbx15+ pluripotent stem cell line under the culture conditions for mouse ES cells. This cell line is very similar to mouse ES cells in cell morphology, growth properties, surface markers, teratoma formation, or the like, while it is different from mouse ES cells in gene expression profiles, DNA methylation patterns, and generation of chimeric animals, so it is named as induced pluripotent stem cells (iPS cells) (Takahashi K, Yamanaka S. Induction of pluripotent stem cells from mouse embryonic and adult fibroblast cultures by defined factors. Cell 2006; 126: 663-676).

In July 2007, the Yamanaka study group further performed screening using Nanog instead of Fbx15 and obtained a Nanog+ iPS cell line. This iPS cell is not only very similar to the mouse ES cell in cell morphology, growth properties, expression markers, and formation of teratoma comprising the histiocyte structures of the three germ layers when being subcutaneously transplanted into the mouse, but also almost identical to the mouse ES cell in DNA methylation patterns, gene expression profiles, chromatin status, and generation of chimeric animals. In addition, the study also found that the reactivation of proto-oncogene c-Myc is responsible for neoplasia occurred in a chimeric animal; but the above four transfected genes are not expressed in the iPS cell. This indicates that these genes play a role only in the induction process and the pluripotent state of the iPS cell is attributable to the expression of endogenous transcription factors such as Nanog gene (Okita K, Ichisaka T et al., germline-competent induced pluripotent stem cells. Nature 2007; 448: 313-317). Another paper published independently by some American scientists at the same time also confirmed that the above four transcription factors are sufficient to make mouse fibroblasts induced and reconstructed in vitro into iPS cells that are similar to mouse ES cells (Wernig M et al., In vitro reprogramming of fibroblasts into a pluripotent ES cell-like state. Nature 2007; 448: 318-324).

It was recently reported that mouse liver cells and stomach epithelial cells can also be reconstructed into iPS cells, and as revealed by genetic cell lineage tracing analysis, iPS cells are derived from the direct reconstruction of somatic cells of fixed lineage and the integration of retrovirus into specific gene loci is not found to be related to nucleus reconstruction (Aoi T et al., Generation of Pluripotent Stem Cells from Adult Mouse Liver and Stomach Cells. Science 2008).

Some researchers also successively obtained iPS cells by introducing the same four transcription factors into human skin fibroblasts by using the same technology. Similarly, primary human fibroblast-like synovial cells as well as cell lines derived from neonatal fibroblasts may also be reconstructed into iPS cells. Such iPS cells are similar to hES cells in cell morphology, proliferation ability, surface antigen markers, gene expression profiles, the epigenetic status of pluripotent stem cell-specific genes, telomerase activity and so on, and can be differentiated into different types of cells of the three germ layers in in vitro culture and in teratoma formation in mice (Takahashi K et al., Induction of pluripotent stem cells from adult human fibroblasts by defined factors. Cell 2007; 131: 861-872). At the same time, the Thomson study group of the University of Wisconsin also reported the successful induction of embryonic fibroblasts into human iPS cells having the essential characteristics of hES cells, only that they used lentivirus as the vector and selected the four factors including Oct4, Sox2, Nanog, Lin28 from 14 candidate genes to perform transduction (Yu J et al., Induced pluripotent stem cell lines derived from human somatic cells. Science 2007; 318: 1917-1920).

Park IH obtained the same results by adopting the strategy of the Yamanaka study group using primary fibroblasts from the skin or lung of fetus, newborn, or adult (including fibroblasts from skin biopsy of one healthy man). They also found that Oct4 and Sox2 are necessary in the course of induction and reconstruction into iPS cells and it is these two transcription factors that maintain the pluripotency of human iPS cells, while Klf4 and c-Myc act to alter the structure of chromatin and thus facilitate the binding of Oct4 with Sox2 to increase the efficiency of induction (Park IH et al., Reprogramming of human somatic cells to pluripotency with defined factors. Nature 2008; 451: 141-146). Furthermore, the significance of this study rests on the induction of fibroblasts from skin biopsy into iPS cells. As indicated by the above study, it is feasible to prepare patient-specific stem cells by extracting somatic cells from biopsied human skin tissues and then inducing the somatic cells. Accordingly, it is hopeful to overcome immunological rejection occurred in cell transplantation therapies. The introduction of c-Myc gene may lead to an incidence of tumor of up to 20% in chimeric mice, which may impede their intending clinical applications (Okita K, Ichisaka T, Yamanaka S. Generation of germline-competent induced pluripotent stem cells. Nature 2007; 448:313-317). In view of this, the Yamanaka study group recently reported that, iPS cells may also be obtained by transfecting mouse and human skin fibroblasts with other three genes except c-Myc under adjusted culture conditions. Although the removal of c-Myc gene can significantly improve the safety of intending clinical applications, iPS cells are generated at significantly reduced efficiency (Nakagawa M et al., Generation of induced pluripotent stem cells without Myc from mouse and human fibroblasts. Nat Biotechnol 2008; 26: 101-106).

Nevertheless, although a great number of methods involving iPS cells have been developed, considering that currently iPS cells suffer the problems including use of virus as gene vector, low efficiency and use of oncogene c-Myc, the optimal scheme is to directly induce somatic cells into iPS cells through drugs, which process and the subsequent differentiation process are performed in a chemically defined medium, thereby obtaining completely safe therapeutic cells. However, it is impossible to predict a drug that can replace some factor based on existing knowledge, and the optical method is high throughput screening. High throughput screening requires a stable iPS induction system with high efficiency, wherein high efficiency is required to reduce aperture and increase throughput at the same cost; stability is required to eliminate difference between batches, because repeatability is very important if millions of drugs are to be screened.

However, the media used in existing iPS induction systems all require serum. Serum is unstable between batches. Furthermore, it contains many uncertain ingredients whose concentrations often vary greatly. As such, serum is inherently disadvantageous with respect to study mechanism. In view of this, the applicants hope to study whether iPS cells can be induced with a serum-free medium.

WO9830679 reported a KnockOut Serum Replacement (KOSR), and a serum-free embryonic stem cell medium containing the same. However, this medium cannot support the proliferation and generation of iPS cells.

To date, there is no literature reporting induction of iPS cells without serum in the whole process in mouse somatic cells, especially fibroblasts that are readily available.

Further, in the prior art, a general method for inducing pluripotent stem cells has an induction efficiency of about 0.01-0.5% in fetal bovine serum on feeder cells in about 14 days (see Qin, D., Li, W., Zhang, J. & Pei, D. Direct generation of ES-like cells from unmodified mouse embryonic fibroblasts by Oct4/Sox2/Myc/Klf4. Cell research 17, 959-962 (2007); Takahashi, K. & Yamanaka, S. Induction of pluripotent stem cells from mouse embryonic and adult fibroblast cultures by defined factors. Cell 126, 663-676 (2006); Meissner, A., Wernig, M. & Jaenisch, R. Direct reprogramming of genetically unmodified fibroblasts into pluripotent stem cells. Nature biotechnology 25, 1177-1181 (2007); Takahashi, K. et al., Induction of pluripotent stem cells from adult human fibroblasts by defined factors. Cell 131, 861-872 (2007); Yamanaka, S. Strategies and new developments in the generation of patient-specific pluripotent stem cells. Cell Stem Cell 1, 39-49 (2007)).

The generating of embryonic stem cells or induced pluripotent stem cells is known from the following documents:
AMIT M ET AL: "Feeder layer- and serum-free culture of human embryonic stem cells", BIOLOGY OF REPRODUCTION, NEW YORK, NY [U.A.] : ACADEM. PRESS, US, vol. 70, 1 January 2004 (2004-01-01), pages 837-845, XP002978624, ISSN: 0006-3363, DOI: 10.1095/BIOLREPROD.103.021147; TROND AASEN ET AL: "Efficient and rapid generation of induced pluripotent stem cells from human keratinocytes", NATURE BIOTECHNOLOGY, vol. 26, no. 11, 1 November 2008 (2008-11-01), pages 1276-1284, XP055019438, ISSN: 1087-0156, DOI: 10.1038/nbt.1503; LUDWIG TENNEILLE E ET AL: "Feeder-independent culture of human embryonic stem cells", NATURE METHODS, NATURE PUBLISHING GROUP, GB, vol. 3, no. 8, 1 August 2006 (2006-08-01), pages 637-646, XP008084296, ISSN: 1548-7091, DOI: 10.1038/NMETH902; US 2009/081784 A1; WO2009/0231 A1; VANESSA DING ET AL: "Deciphering the Importance of Three Key Media Components in Human Embryonic Stem Cell Cultures", BIOTECHNOLOGY LETTERS, SPRINGER NETHERLANDS, DORDRECHT, vol. 28, no. 7, 1 April 2006 (2006-04-01), pages 491-495, XP019231244, ISSN: 1573-6776, DOI: 10.1007/S10529-006-0005-8; and WO 2006/044204 A2.

Thus, the present invention provides a serum-free medium for inducing pluripotent stem cells in the absence of feeder cells in a quicker manner with higher efficiency than the prior art.

### SUMMARY OF THE INVENTION

In a first aspect, the present invention provides a serum-free medium for culturing cells.

This serum-free medium for inducing pluripotent stem cells comprises a basal medium, a serum replacement additive formulated by KnockOut Serum Replacement (KOSR) and N2-supplement, leukemia inhibitory factor (LIF), and one or more receptor tyrosine kinase growth factors selected from at least one of bFGF, EGF, IGF2, or VEGF, wherein the bFGF has a concentration of 3 ng/mL to 20 ng/mL in the final medium.

In another aspect, the present invention provides a method for inducing pluripotent stem cells from somatic cells with high efficiency using the above serum-free medium, comprising:
(a) introducing one or more stem cell pluripotent factors into somatic cells;
(b) culturing the resulting somatic cells in (a) in the serum-free medium of the present application under conditions suitable for cell growth so as to induce and reprogram the somatic cells into pluripotent stem cells;
(c) detecting and analyzing the induced cells for pluripotency;
(d) picking up pluripotent monoclones of the induced pluripotent stem cells;
(e) culturing the monoclone cells in (d) under conditions suitable for growth of embryonic stem cells.

The present invention further relates to a method for inducing pluripotency, which, in addition to the above steps of (a)-(e), further comprises introducing a reporter gene into the somatic cells, and prior to the step (c), indicating the generation of pluripotent stem cells and detecting the efficiency thereof first through the reporter gene.

In still another aspect, the present invention further relates to the uses of the serum-free medium of the present invention in quickly inducing and reprogramming somatic cells into pluripotent stem cells with high efficiency.

Finally, the present invention further relates to the uses of the medium of the present invention in screening compounds, especially high throughput screening compounds using induced pluripotent stem cells in the iPS system.

### BRIEF DESCRIPTION OF THE DRAWINGS

Fig. 1: MEF cells infected with four factors (4F, Oct4, Sox2, Klf4, and c-Myc) cannot grow in mKSR medium and cannot produce iPS colonies. The control is iPS cells cultured in a serum medium (mES) using a known classical method. Scale, 100 µm.
Fig. 2: Serum-free iPS-SF1 can support the generation of iPS cells better than a stem cell medium with serum.
   a. Growth curves of MEF cells in FBS medium and iPS-SF1 medium (n = 3).
   b. FACS analysis for Oct-GFP positive cells was performed on MEF cells infected with four factors (4F, Oct4, Sox2, Klf4, and c-Myc) or three factors (3F, Oct4, Sox2, and Klf4) at different time points. The infected cells were cultured in mES medium and iPS-SF1 medium respectively. N = 2; error bar indicates s.d.
   c. MEF cells infected with the four factors were cultured in mES medium and iPS-SF1 medium respectively. Six days later, it was found that Oct4-GFP which indicates cells are reprogrammed into pluripotent cells was only expressed in the cells cultured in iPS-SF1 medium and clone morphology was formed, while Oct4-GFP was not expressed in the mES cells cultured in classical serum medium mES. Scale, 100 µm.

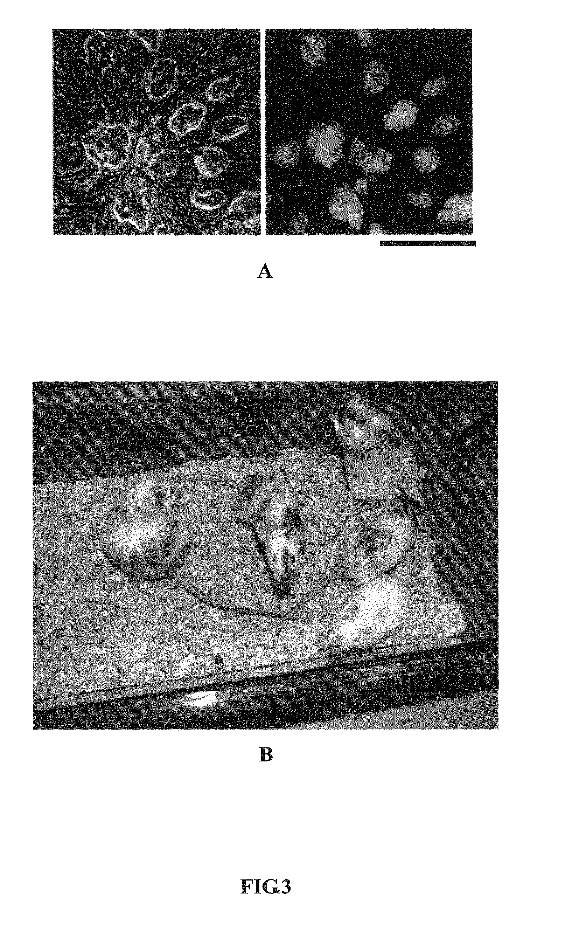

Fig. 3: The induced pluripotent stem cell (iPS) line obtained with the serum-free medium iPS-SF1 has pluripotency.
   a. The iPS cell line obtained from iPS-SF1 culture after infection has protuberant clone morphology similar to typical mouse stem cells and strongly expressed Oct4-GFP indicating pluripotency.
   b. The iPS cell line obtained from iPS-SF1 culturing after infection can give rise to a chimeric mouse after being injected into a blastula. The donor blastula is a white ICR mouse and black hair on the mouse indicates that chimerism has happened, which demonstrates that the iPS cell line established by iPS-SF1 culture is capable of involving in normal *in vivo* development.

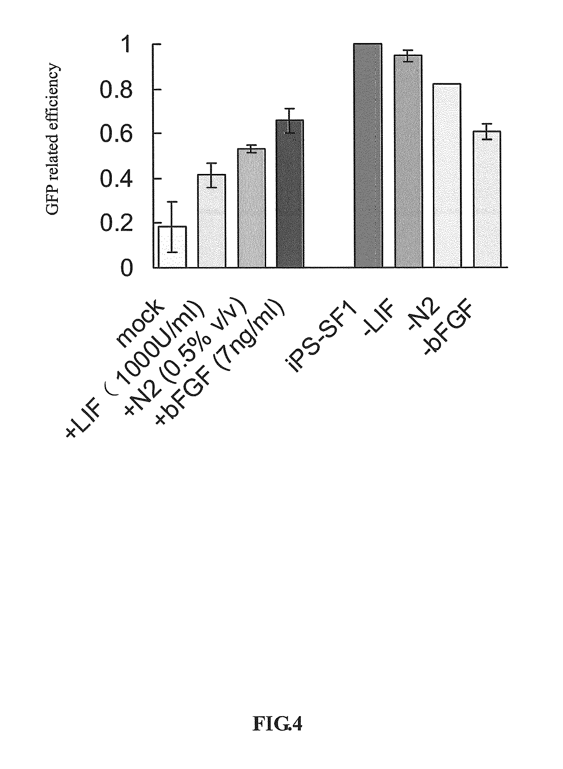

Fig. 4: Under eight different culture conditions and at day 7 after infection, the percentage of Oct4-GFP positive cells was measured when being infected with the four factors, as described above. The value of the iPS-SF1 sample is set to be 1. The left four bars indicate the positive effects produced when the three supplements are added respectively into iPS-SF1. The Mock medium is supplemented with DMEM containing 10% KOSR. The right four bars indicate the negative effects produced after each of these components is removed from iPS-SF1.

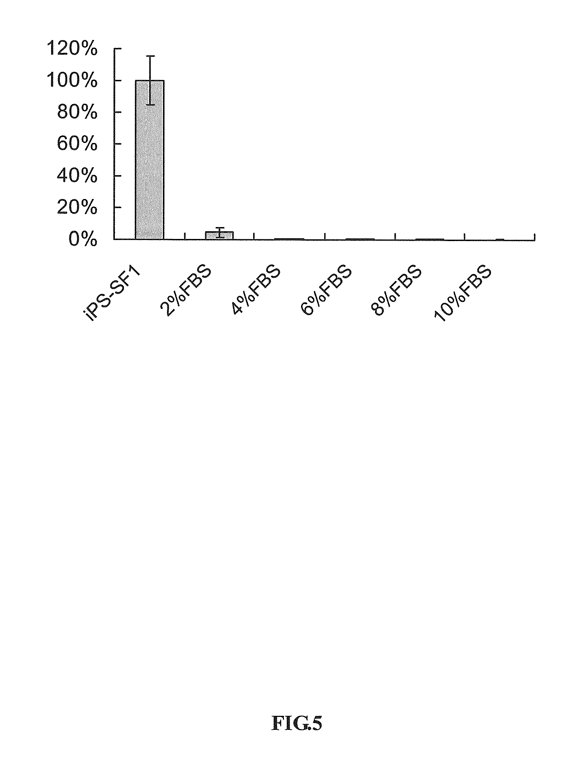

Fig. 5: Serum is a strong reprogramming inhibitor. For the MEF cells infected with the four factors, the percentage of Oct4-GFP positive cells was analyzed at day 7 after infection. Serum was supplemented into iPS-SF1 in different concentrations. N = 2; error bar indicates s.d.

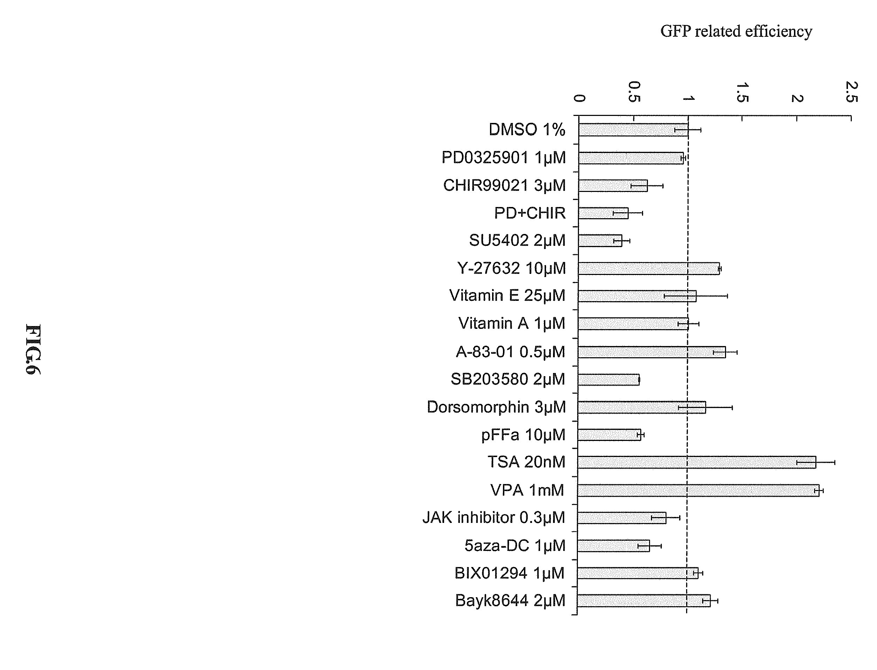

Fig. 6: With iPS-SF1 as the screening tool, the target compounds for common signaling pathways were selected. The dotted line shows the value of the control sample which is set to be 1. n = 2; error bar indicates s.d.

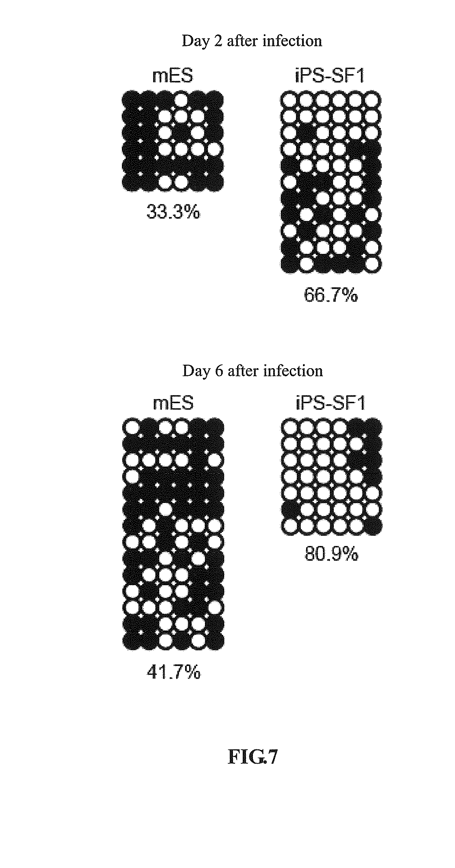

Fig. 7: Analysis on the methylation of Nanog promoter in the iPS process mediated by three factors Oct4/Sox2/Klf4. Fibroblasts infected with the three factors were cultured in mES (medium with serum) and iPS-SF1 respectively. Samples were taken at day 2 and day 6 after infection to analyze the methylation status of Nanog promoter using the bisulfate sequencing method, wherein white circles represent unmethylated CpG nucleotide pairs, while black circles represent methylated CpG nucleotide pairs.

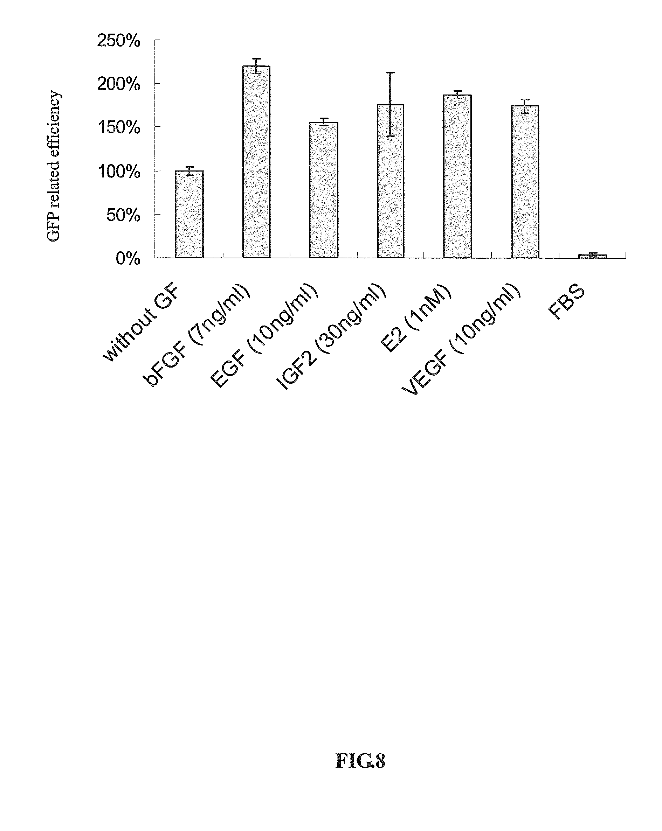

Fig. 8: Effects of different growth factors (including tyrosine kinase and estrogen) in reprogramming. The growth factors shown in the figure were added respectively into iPS-SF1 without any growth factor, and Oct4-GFP was analyzed at day 7 after infection, wherein E2 is an estrogen and the FBS medium is used as the negative control. n = 2; error bar indicates s.d.

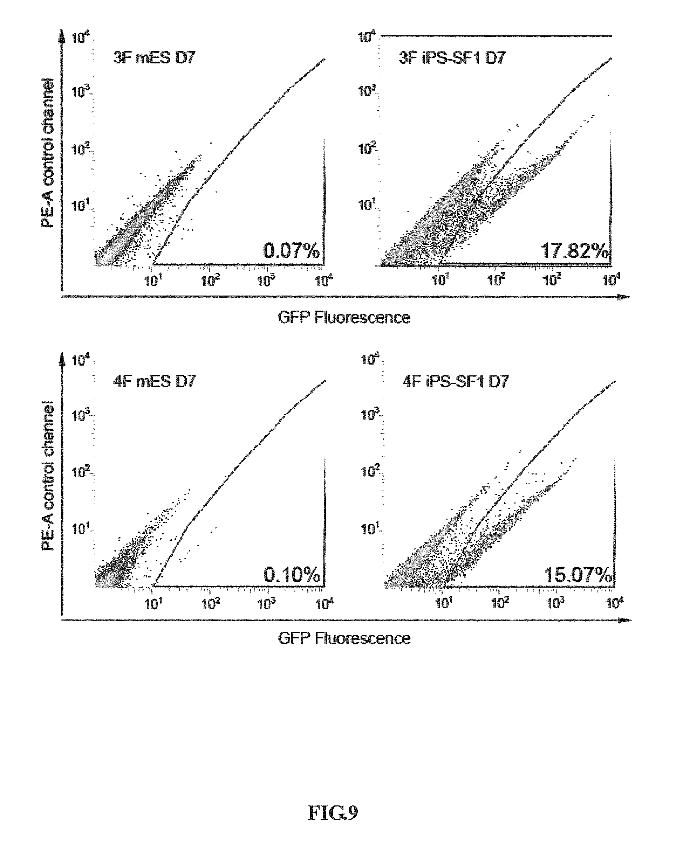

Fig. 9: Representative FACS graphs of infected MEF cells cultured in different media. The signal from the PE pathway is used as the autofluorescent control. For iPS-SF1, the Oct4-GFP colonies were significantly improved at day 7 after infection both in MEF cells infected with the four factors and in MEF cells infected with the three factors.

### DETAILED DESCRIPTION OF THE INVENTION

### Definitions and Technologies

Unless otherwise indicated, the present invention will be practiced using traditional technologies of molecular biology, microbiology, cell biology, immunology, and recombinant DNA, which fall into the technical field of the art. See, for example, Sambrook, Fritsch, and Maniatis, Molecular Cloning: A Laboratory Manual, Third Edition (2002); CURRENT PROTOCOLS IN MOLECULAR BIOLOGY (F. M. Ausubel et al. (Eds) (1987)); Series METHODS IN ENZYMOLOGY (Academic Press, Inc.): PCR2: A PRACTICAL APPROACH (M.J. MacPherson, B.D. Hames and GR. Taylor (Eds), (1995)), Harlow and Lane (Eds), (1988) ANTIBODIES, A LABORATORY MANUAL and ANIMAL CELL CULTURE (R.I. Freshney (Editor), (1987)); W. French Anderson et al., HANDBOOK OF STEM CELLS, Vol. 2.

Unless otherwise stated, all the terms used herein have meanings conventionally perceived by those skilled in the art. For an easy understanding of the present invention, some terms used herein are defined as follows.

As used in the specification and claims, the singular form "a," "an" and "the" includes plural references unless the context clearly dictates otherwise. For example, the term "a cell" includes a plurality of cells, including mixtures thereof.

All numerical designations, e.g., pH, temperature, time, concentration, and molecular weight, including ranges, are approximations. It is to be understood, although not always explicitly stated, that the term "about" shall be provided in front of all numerical designations. It is also to be understood, although not always explicitly stated, that the reagents described herein are merely exemplary and that equivalents of such reagents are known in the art.

"Induced pluripotent stem cells (iPS cells)" described herein are such cells which are derived from somatic cells through in vitro induction of somatic cells by stem cell pluripotent factors capable of inducing the reprogramming of somatic cells. Under ES cell culture conditions, iPS cells are very similar to mouse ES cells in cell morphology, growth properties, surface marker expression, formation of teratoma comprising the histiocyte structures of the three germ layers when being subcutaneously transplanted, and also are almost identical to mouse ES cells in DNA methylation patterns, gene expression profiles, chromatin status, and generation of chimeric animals, or the like.

The term "basal medium" used in the present invention refers to any medium that can support cell growth. The basal medium provides standard inorganic salts such as zinc, iron, magnesium, calcium, and potassium, vitamins, glucose, buffer system, and key amino acids. The basal medium that can be used in the present invention includes, but is not limited to, Dulbecco's Modified Eagle's Medium (DMEM), Minimal Essential Medium (MEM), Basal Medium Eagle (BME), RPMI 1640, F-10, F-12, α Minimal Essential Medium (αMEM), Glasgow's Minimal Essential Medium (G-MEM), and Iscove's Modified Dulbecco's Medium. Those skilled in the art have already known how to select a suitable basal medium for the cells to be cultured. In a preferred embodiment, the basal medium is a mixture of DMEM and F12 (1:1) or DMEM with high glucose. In a more preferred embodiment, the basal medium is DMEM with high glucose (e.g. 4.5 g/L).

As used in the present invention, the term "serum replacement additive" refers to, in cell culture, an additive that is added into the basal medium to replace partly or wholly the role of serum in supporting cell survival or growth. Generally, it includes insulin, transmetalloprotein, trace element, vitamin, or other factors. These factors are generally not included in the basal medium but are provided by serum used generally in culturing cells. The serum replacement additive comprises at least one or more of the following components that support cell growth: one or more insulins or replacements thereof, one or more transmetalloproteins or replacements thereof, one or more trace elements, one or more vitamins, one or more amino acids, one or more hormones or hormone-like compounds, serum albumin or replacements thereof, and one or more lipids. Many commercialized serum replacement additives, such as KnockOut Serum Replacement (KOSR), N2, B27, Insulin-Transferrin-Selenium Supplement (ITS), and G5 are well known and are readily available to those skilled in the art. These additives are characterized by well-defined ingredients, so the concentrations of its components can be determined based on its proportion in the medium.

Those skilled in the art can readily prepare a serum replacement additive based on the prior art, the type of cells to be cultured, and other aspects. The serum replacement additive used herein is a mixture additive obtained by mixing KOSR and 10 N2 in certain ratio. Most preferably, in the final medium, KOSR has a concentration of 10%, and N2 has a concentration of 0.5%.

The receptor tyrosine kinase used in the present invention includes receptor tyrosine kinase growth factors having the properties of receptor tyrosine kinases and is selected from bFGF, EGF, IGF2, or VEGF. Most preferably, the receptor tyrosine kinase is bFGF. Based on the type of cells to be cultured and other conditions, those skilled in the art may readily determine the sufficient concentration of the receptor tyrosine kinase to be added to maintain cell growth. The concentration is about 3-20 ng/mL, more preferably about 5-15 ng/mL, and most preferably about 7 ng/mL. In a preferred embodiment, the medium of the present invention may comprise about 5 ng/mL bFGF, 10 ng/mL EGF, 25 ng/mL IGF2 and/or 10 ng/mL VEGF. In the most preferred embodiment, the medium of the present invention comprises about 5 ng/mL bFGF.

As used herein, LIF refers to leukemia inhibitory factor which is a growth factor that is often added in culturing stem cells as known in the art. Those skilled in the art can adjust the concentration of LIF used in a specific medium based on specific conditions. In the medium of the present invention, the concentration of LIF added in the medium is preferably in the range of 500 U/mL to 2000 U/mL, more preferably in the range of 700 U/mL to 1400 U/mL, and most preferably about 1000 U/mL.

As known by those skilled in the art, in order to facilitate cell growth, other components may optionally be added in the medium. Those skilled in the art have already known how to select other components (such as L-glutamine, NEAA MEM, and 2-mercaptoethanol) required by a specific medium based on cells to be cultured and other conditions.

The serum-free medium according to the present invention is prepared by conventional technologies known by those skilled in the art, for example, the technologies and conditions for mix preparation of serum as described in A LABORATORY MANUAL and ANIMAL CELL CULTURE (R.I. Freshney (Editor), 1987); W. French Anderson et al., HANDBOOK OF STEM CELLS.

The term "somatic cell" used herein is a concept which is opposite to "germ cell" and "embryonic stem cell". Somatic cells are cells that are generated by differentiation of "embryonic stem cells" and do not possess pluripotency any more but have a specific function. Specifically, somatic cells are cells that are generated by differentiation of "embryonic stem cells" or further development of the inner cell masses and do not possess pluripotency any more but have a specific function. Generally, somatic cells are taken from fetal mice that have passed the blastula stage (for mice, specifically, being 3.5 days after fertilization) with regard to the development stage or from adult mice. In taking the cells, possibly pluripotent germ cells and their sources (such as primitive spermatogonium, genital ridge stem cells) shall be generally avoided. The somatic cells used herein are preferably derived from mammals, more preferably from human, monkey, dog, cat, rat, or mouse, and most preferably from mouse. The somatic cells herein may be any type of somatic cells in the organism, preferably fibroblasts or meningocytes.

The "stem cell pluripotent factor capable of inducing the reprogramming of somatic cells" described herein is a factor critical for maintaining the pluripotency of stem cells. Introducing the factor into somatic cells may induce and reprogram somatic cells into embryonic stem cells under certain conditions. To date, numerous literatures have reported many such factors used for reprogramming, see, for example, Qin, D., Li, W., Zhang, J. & Pei, D. Direct generation of ES-like cells from unmodified mouse embryonic fibroblasts by Oct4/Sox2/Myc/Klf4. Cell research 17, 959-962 (2007); Okita, K., Ichisaka, T. & Yamanaka, S. Generation of germline-competent induced pluripotent stem cells. Nature 448, 313-317 (2007); Wernig, M. et al., In vitro reprogramming of fibroblasts into a pluripotent ES-cell-like state. Nature 448, 318-324 (2007); Yamanaka, S. Strategies and new developments in the generation of patient-specific pluripotent stem cells. Cell Stem Cell 1, 39-49 (2007), and so on. There are many stem cell pluripotent factors known by those skilled in the art which can be used for such purposes. Preferably, the pluripotent factors include Oct4, Sox2 (optionally Sox1), C-myc (optionally L-Myc or N-Myc), Klf4 (optionally Klf5 or Klf2), Esrrb, Nanog, and Lin28. The pluripotent factors described above may be derived from any source depending on the cells into which they are to be introduced, and preferably, are mouse pluripotent factors and variants thereof, such as Sox2 (NCBI accession No.: NM_011443.3, mouse SRY-box containing gene 2); Oct4 (NCBI accession No.: NM_013633.2, mouse POU domain, class 5, transcription factor 1 (Pou5f1)); Klf4 (NCBI accession No.: NM_010637.2, mouse Kruppel-like factor 4); c-Myc (NCBI accession No.: NM_010849.4, mouse myelocytomatosis oncogene) (c-Myc) ; Sox1 (NCBI accession No.: NM_009233.3, mouse SRY-box containing gene 1); Klf2 (NCBI accession No.: NM_008452.2, mouse Kruppel-like factor 2 (lung)); Klf5 (NCBI accession No.: NM_009769.4, mouse Kruppel-like factor 5); Nanog (NCBI accession No.: NM_028016); or Lin28 (NCBI accession No.: NM_145833). C-Myc may also be replaced by its mutants L-myc (accession No.: NM_008506.2, mouse v-myc myelocytomatosis viral oncogene homolog 1v, lung carcinoma derived (avian)) (Mycl1); or N-Myc (accession No.: NM_008709.3, mouse v-myc myelocytomatosis virus related oncogene, neuroblastoma derived (avian)) (Mycn).

The term "inducing and reprogramming" (sometimes also briefed as "inducing") described herein refers to a process of dedifferentiating somatic cells into pluripotent stem cells. Preferably, somatic cells may be induced to dedifferentiate into pluripotent stem cells by introducing pluripotent factor cDNA required for maintenance of stem cell pluripotency into somatic cells (Takahashi K, Yamanaka S. Cell. 2006; 126:663-676; Wernig M, Meissner A, Foreman R, et al., Nature. 2007; 448: 318-324; Yu J. Vodyanik MA, Smuga-Otto K et al., Science. 2007; 318: 1917-1920). Among others, preferably, the pluripotent factor includes one or more of Oct4, Sox2 (optionally Sox1), C-myc (optionally L-Myc or N-Myc), Klf4 (optionally Klf5 or Klf2), Nanog, and Lin28.

The methods for introducing the stem cell pluripotent factor cDNA into somatic cells may be a number of technologies known by those skilled in the art, including viral infection, liposome transfection, transposon-mediated insertional expression, membrane-spanning protein, drug induction, electroporation, particle bombardment, and other methods that introduce DNA into cells. Preferably, a viral vector which contains cDNAs is used for transfection. The viral vector includes a variety of viral vectors such as lentiviral vector and retroviral vector, and retroviral vector (such as pMX vector) is preferred, as described in the Examples.

"Conditions suitable for cell growth" as described herein are conventional conditions for culturing stem cells in the art and include some modifications that are suitable for specific cell lines but do not influence the basic properties of the cells. For culture methods and conditions, see W. French Anderson et al., HANDBOOK OF STEM CELLS, Vol. 2.

The reporter gene described herein refers to be capable of indicating that a cell has been transformed through externally applied induction to reach a stage similar to embryonic stem cell, including introduction of a sequence which expresses fluorescent protein or resistance by means of transgene or homologous recombination. This sequence is under the control of the promoters for some genes specifically expressed by embryonic stem cells. Therefore, the expression of this fluorescent protein or resistance gene can be activated when this cell reach embryonic stem cell state such that this cell can possess some detectable characteristics (such as glowing green) to differ from other cells that are not reprogrammed to this state. Common reporter genes used in the art include green fluorescent protein, resistance genes such as ampicillin resistance gene, or the like. Those skilled in the art may select reporter genes suitable for various embodiments based on the culture conditions and uses of cells. See, for example, Young II Yeom et al., Germline regulatory element of Oct-4 specific for the totipotent cycle of embryonal cells, Development 122,000-000 (1996), Printed in Great Britain, The company of Biologists Limited 1996, P881-894; Shin-ya Hatano et al., Pluripotential competence of cells associated with Nanog activation, Mechanisms of Development 122(2005), 67-79.

The methods for detecting cell pluripotency as described herein are well known by those skilled in the art. See, for example, Yamanaka, S. Strategies and new developments in the generation of patient-specific pluripotent stem cells. Cell Stem Cell 1, 39-49 (2007), and so on. The methods include identification of the expression of pluripotent molecular marker, detection of methylation status of cells, formation of embryonic body (EB), formation of teratoma, generation of chimeric mouse using induced pluripotent stem cells, and so on.

"Chimeric mouse" as described herein is implemented through "chimeric mouse" technology well known by those ordinarily skilled in the art. This involves injecting embryonic stem cells or the iPS cells obtained through the technology described herein into a mouse blastula to mix with the embryonic cells therein and then grow and develop together in the uterus of the surrogate mother mouse. After birth, the mouse has its tissues composed of these two embryonic cells together in admixture, just like mosaic patterns. Such a mouse is called as a chimeric mouse (Evans M J, et al.; The ability of EK cell to form chimeras after selection of clones in G418 and some observation on the integration of retroviral vector proviral DNA into EK cells [M]; Cold Spring Harbor Symposia on Quantitative Biology; 1985; Xian MW, Wu BY, Hu XL, Shang KG, Wu HL, 1996. Construction of chimeric mice of ES cells by microinjection method. Hereditas (Beijing) 18(1): 7-10 (in Chinese)). The most direct and critical evidence to verify whether iPS cells and embryonic stem cells have similar properties rests on whether iPS cells can lead to the formation of chimeric mice.

"Screening compounds" as described herein means screening in a specified compound library, through verifying the characteristics of reporter gene or the cell itself, for: 1. compounds that affect the iPS process and induction conditions; 2. compounds capable of replacing some or all currently known factors required by the iPS induction process. Those skilled in the art have developed methods for screening compounds using the iPS process, see for example, Yan Shi et al., Induction of Pluripotent Stem Cells from Mouse Embryonic Fibroblasts by Oct4 and Klf4 with Small-Molecule Compounds, Cell Stem Cell 3, 568-574, Nov. 6, 2008.

The "high throughput" screening method as described herein is known by those skilled in the art, wherein the role of each individual in a large library in a specified test model can be screened in a short period of time using automated instruments and small sample size. See, for example, Nil Emre, et al., A chemical approach to stem cell biology, Current Opinion in Chemical Biology 2007, 11: 252-258. In this method, high throughput screening is performed for the iPS process in compound libraries and natural product libraries of large order of magnitude.

### Examples

The standard laboratory practices of the inventors are illustrated in the following examples which are used to exemplify the mode of the present invention. The scope of the present invention shall not be construed as being limited to these examples. According to what is disclosed herein and the common level of those skilled in the art, it shall be appreciated that the following examples are only for illustration and may be subjected to various changes, modifications, and alterations without departing from the scope of the present invention. Unless otherwise stated, all the technologies concerned are conventional technologies in molecular biology, cellular biology, biochemistry, or other fields as well known by those skilled in the art.

### Summary of technologies used in the present invention:

Unless specifically stated, all the substances mentioned herein are from Invitrogen.

### Cell culture:

Mouse embryonic fibroblasts were derived from the embryo at e13.5 of hemizygotes for Oct-GFP transgene allele and Rosa26 allele, and were cultured in the fibroblast medium below: DMEM with high glucose, supplemented with 10% FBS, L-glutamine, and NEAA. Both iPS cells and ES cells were cultured on the MEF feeder of a serum-containing medium (mES) and a serum-free medium (mKSR). The designation and composition of individual media herein are shown in Table 1 in detail. MEF feeder cells had been deactivated by mitomycin C.

**Table 1: Media used herein**

| Compos ition | Design ation | mES | mKSR | FB-SF1 | iPS-SF1 |
|---|---|---|---|---|---|
| Basal medium | | DMEM with high glucose | Knock Out DMEM | DMEM/F12 (1:1) | DMEM with high glucose |
| Serum or serum-replacemen t additive | | 15% FBS | 10% KOSR | 10% KOSR | 10% KOSR |
| | | | | 0.5% N2 | 0.5% N2 |
| Growth factor | | 1000U/mL LIF (Millipore) | 1000U/mL LIF | 1000U/mL LIF 5ng/mL bFGF | 1000U/mL LIF 5ng/mL bFGF |
| Other components | | 2mM L-glutamine 1/100 NEAA MEM 0.1mM 2-mercaptoeth anol 1mM sodium pyruvate Penicillin/strep tomycin (Hyclone) | 2mM L-glutamine 1/100 NEAA MEM 0.1mM 2-mercaptoeth anol Penicillin/strep tomycin | 2mM L-glutamine 1/100 NEAA MEM Penicillin/strep tomycin | 2mM L-glutamine 1/100 NEAA MEM 0.1mM 2-mercaptoeth anol Penicillin/strep tomycin |

### Production of retroviruses and generation of iPS cells:

The retroviral vectors (pMXs) for DNAs comprising mouse Oct4, Sox2, Klf4, and c-Myc were purchased from Addgene. Generation and infection of viruses were performed according to existing technology (Qin, D., Li, W., Zhang, J. & Pei, D. Direct generation of ES-like cells from unmodified mouse embryonic fibroblasts by Oct4/Sox2/Myc/Klf4. Cell research 17, 959-962 (2007); Qin, D. et al., Mouse meningiocytes express Sox2 and yield high efficiency of chimeras after nuclear reprogramming with exogenous factors. J Biol Chem 283, 33730-33735 (2008)). In brief, these plasmids were transfected into PlatE cells using conventional methods; the viral supernatant was then collected and filtered for 48 hours in order to infect MEF cells, wherein polybrene was supplemented. In day 2, the same procedure was repeated. The day at which the viral supernatant is removed is defined as day 0 after infection. The fibroblasts infected with viruses (i.e. transfected with Oct4, Sox2, Klf4 and/or c-Myc; hereinafter, unless specially stated, three-factor infection represents infection with Oct4, Sox2, and Klf4, and four-factor infection represents infection with Oct4, Sox2, Klf4, and c-Myc) were cultured in the medium of the present invention, and 10-15 days after infection, iPS colonies were picked up based on Oct-GFP (that is, colonies emitting fluorescence under a fluorescence microscope) and typical ES morphology. Afterwards, the picked-up colonies were expanded and maintained like ES cells (ut supra, Qin, D., Li, W., Zhang, J. & Pei, D, 2007; and Qin, D. et al., 2008).

### Quantification of reprogramming efficiency:

The main method for quantifying reprogramming efficiency is FACS analysis for Oct4-GFP positive cells. Based on the results of the timing process, the infected MEF cells were digested with trypsin at days 7 and 9 after infection and then analyzed through FACSCalibur. GFP positive cells received the gates of control signals from PE pathway, and 15,000 events were recorded as a minimum. Cells infected with pMXs-FLAG were used as negative control. In order to verify the efficiency as analyzed by FACS, GFP positive colonies were counted directly under a fluorescence microscope at day 14 after infection.

### Characterization of iPS cells:

Alkaline phosphatase staining and immunofluorescence staining were performed (ut supra, Qin, D., Li, W., Zhang, J. & Pei, D, 2007; and Qin, D. et al., 2008). The following primary antibodies were used: mouse anti-Oct4 (Santa Cruz), mouse anti-SSEA1 (Abcam), and mouse anti-Nanog (Abcam).

### Example 1: The traditional mKSR medium cannot maintain the growth of MEF cells transfected with the four factors, and meanwhile cannot induce the formation of iPS cells.

As described above, the viruses of the four factors were mixed in 1:1:1:1 (1 mL each) and then infected into totally 35,000 fibroblasts in one well of a six-well plate and cultured in mKSR medium and mES medium respectively at 37°C with 5% CO₂.

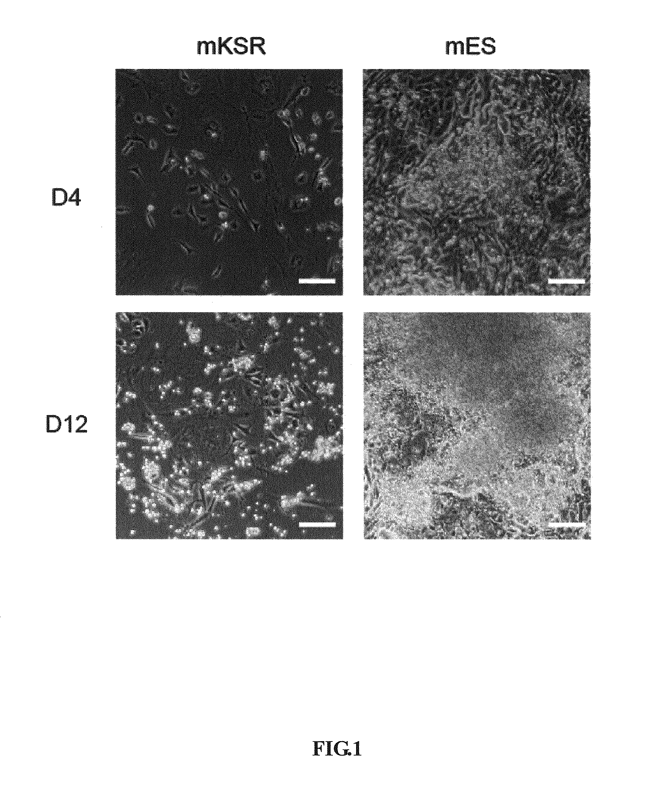

As shown in Fig. 1, the fibroblasts transfected with the four factors and cultured in mKSR grew slowly and still did not form colony morphology of pluripotent cells, indicating that mKSR medium cannot induce and generate iPS colonies.

### Example 2: Use of iPS-SF1 medium can greatly increase the efficiency of inducing iPS cells.

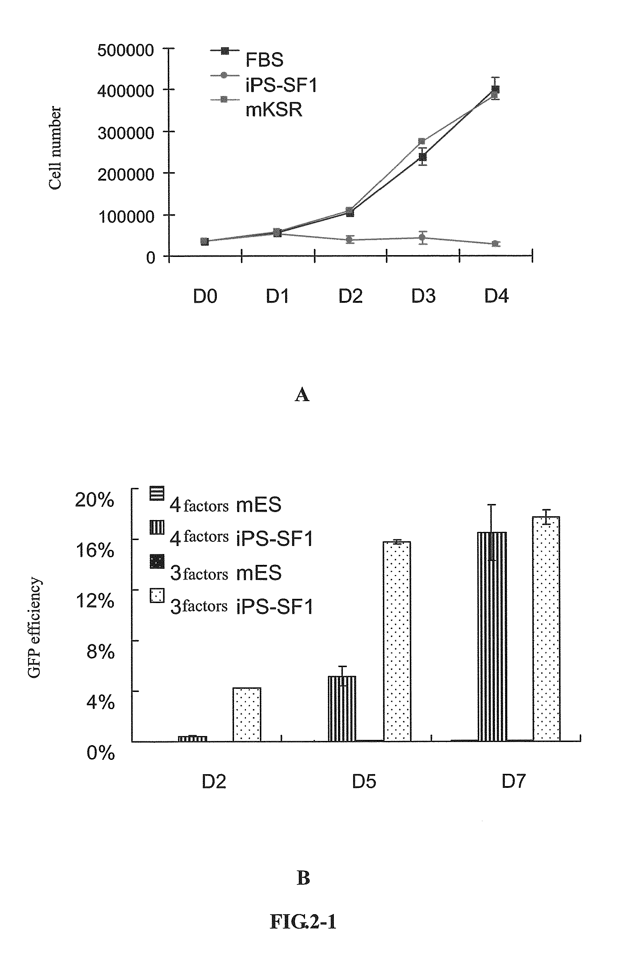

A. MEF cells were cultured in FBS medium and iPS-SF1 medium respectively. The growth curves thereof are almost identical, indicating that iPS-SF1 has no effect on MEF cell culture. In contrast, the growth of MEF cells substantially stopped in mKSR (Fig. 2A).

After three factors (Oct4, Klf4, and Sox2) or four factors (cMyc, Oct4, Klf4, and Sox2) were transfected into fibroblasts, the transfected cells were cultured in iPS-SF1 medium respectively and the efficiency of GFP positive cells was measured as described above at days 2, 5, and 7 after transfection. It was observed that all cells, whether being transfected with the four factors or the three factors, exhibited high efficiency (Fig. 2B) and even reached an efficiency of 18% at day 7. Thus, this is a great improvement as compared to traditional methods (mES control as shown).

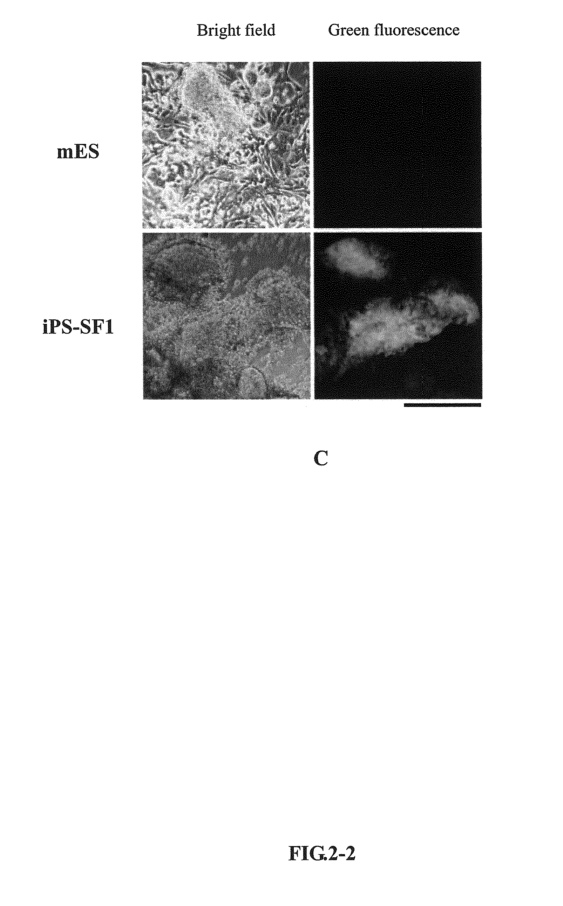

Accordingly, in this process, direct observation using a fluorescence microscope could also find that, for the transfected cells cultured in iPS-SF1 medium, ES-like clones strongly expressing Oct4-GFP appeared at day 6 after transfection, while the clones formed in the traditional medium did not express fluorescence (Fig. 2C).

Similarly, as shown in Fig. 9, MEF cells infected with the three factors and the four factors were respectively harvested at day 7 and then analyzed for efficiency by FACS, which indicates that the iPS reprogramming efficiency (which as described above, is represented by the percentage of Oct4-GFP positive cells in overall cells) of the infected MEF cells is much higher than that in the conventional mES medium (iPS-SF1 vs mES: three factors: 0.07% vs 17.82%; four factors: 0.10% vs 15.07%).

### Example 3: iPS cells induced by iPS-SF1 possess pluripotency.

### A. The cell morphology of the iPS cell line obtained through induction is similar to that of the embryonic stem cell.

As described above, fibroblasts were infected with the three factors and then cultured in iPS-SF1 medium all the time. At days 12-14 after infection, representative clone clumps were picked up based on clone morphology and fluorescence expression, and uniform iPS cell lines were formed after several generations of stable passage.

As shown in Fig. 2A, the left panel shows normal embryonic stem cells and the right panel shows the iPS cell lines described above. These cell lines are very similar to embryonic stem cells in morphology and strongly express the green fluorescence of Oct4-GFP.

B. The formation of chimeric mice confirms that the iPS cell lines obtained through induction possess pluripotency.

### Construction of chimeric mice

Blastulas for injection were taken from four-week-old superovulation ICR female mice (white). These mice were housed with male mice of the same strain. 3.5 days after occurrence of an ejaculatory plug (the day the plug is observed is 0.5 day), embryos were collected from the uterus and the fallopian tube, transferred to M16 culture droplets overlaid by paraffin oil, and incubated in an incubator at 37°C with 5% CO₂.

Three hours before injection, iPS cells for chimerism (i.e. the iPS cell lines obtained above) were provided with fresh culture medium instead, digested with trypsin, and then prepared into single cell suspension for later use. Appropriate amounts of iPS cell suspension and embryos at the blastula stage were transferred into M2 injection droplets, the blastula was fixed with a holding pipette under the microinjection system, and iPS cells were drawn with an injection pipette, wherein the pipette was inserted at the feeder far away from the inner cell mass, and each blastula was injected with 10-12 cells. The blastocoele disappeared after injection. The blastula was incubated for 1-3 hours in the culture medium in the incubator, and after the blastocoele recovered, transferred into the uterus of pseudopregnant mother mice that had mated for 2.5 days to grow therein.

Fig. 3-B shows the chimeric mice constructed with iPS cells obtained using the method of the present invention, wherein the non-chimeric mice are pure white, while the chimeric mice exhibit black alternating with white body color because the iPS cells injected therein are sourced from black OG2/Rosa26 mice. This demonstrates that iPS cells obtained according to the method of the present invention can involve in normal *in vivo* development and generate chimeric mice, indicating that these cells do not differ from embryonic stem cells in development.

### Example 4: The medium of the present invention accelerates the demethylation of the promoters for pluripotent genes in the iPS induction process.

Genomic DNAs (700 ng) were acquired from the cell lines (prepared as describe above) infected with the three factors Oct4, Sox2, and Klf4 and cultured in mES medium and the iPS-SF1 medium of the present invention, and exposed to a mixture of 50.6% sodium bisulfite (Sigma S-1516) and 10 mM hydroquinone (Sigma H-9003) overnight so as to be modified by bisulfite. The promoter region of Nanog was amplified by PCR using the primer pair set forth in Table 2. The PCR product was cloned into flat pMD8-T vector (Takara), proliferated in DH5α, and sequenced.

**Table 2**

| Gene | Forward primer (5'-3') | Reverse primer (5'-3') |
|---|---|---|
| Meth-Nanog | AATGTTTATGGTGGATTTT GTAGGT | CCCACACTCATATCAATATAAT AAC |

In the course of inducing iPS cells, ultimately, it needs to activate endogenously expressed pluripotent genes so as to spontaneously maintain the self-renewal of iPS cells independent of exogenous expression. Accordingly, reprogramming is accompanied by demethylation of the promoters for pluripotent genes. In this example, MEF cells were infected with three viruses Oct4, Sox2, and Klf4 in 1:1:1 and cultured in the traditional mES medium and the iPS-SF1 medium of the present invention respectively. At days 2 and 6 after infection, cell samples were taken, and genomic DNAs were extracted, cleaved with DNAase and then treated with bisulfate overnight, such that C (cytosine) in the unmethylated CpG base pair changed to U (uracil). Afterwards, Nanog promoter was produced through proliferation using nested PCR and then delivered to a sequencing company known in the art (such as Invitrogen) for sequencing. Based on the sequencing results, the changes in methylation of Nanog promoter in the iPS process were analyzed.

As shown in Fig. 7, for MEF cells infected with the three factors, Nanog promoter was demethylated slowly in mES (D2: 33%, D6: 41.7%), while in the medium of the present invention (iPS-SF1), this demethylation proceeded rapidly and reached 66.7% at day 2, and then proceeded continuously and reached 80.9% at day 6. This indicates that the fragment detected in Nanog promoter has been substantially activated. The activation of the typical stem cell pluripotent factor Nanog indicates the induction of iPS cells.

As can be seen from the above example, the iPS-SF1 medium of the present invention can successfully induce the reprogramming of iPS cells. Furthermore, this induced reprogramming can avoid infection by carcinogenic c-Myc gene. In addition, for the iPS-SF1 medium of the present invention, its reprogramming efficiency and demethylation speed are much higher than those of existing mES medium.

### Example 5: Effects of individual components of the medium of the present invention on iPS reprogramming efficiency

LIF, N2, and bFGF in iPS-SF1 medium as listed in Table 1 were deleted respectively, or N2 and bFGF were added into DMEM + 10% KOSR (i.e., containing no growth factor but containing NEAA, L-Glutamine or other ingredients at the same time, called as bKSR hereinafter) medium at concentrations equivalent to that of iPS-SF1 respectively, in order to detect the effects of LIF, N2, bFGF on the iPS reprogramming efficiency of MEF cells transfected with the four factors. The signs in Fig. 4 have the following meanings:
Mock: bKSR
Mock + LIF: bKSR + LIF
Mock + N2: bKSR-LIF + N2
Mock + bFGF: bKSR-LIF + bFGF
iPS-SF1 - LIF: LIF-free iPS-SF1
iPS-SF1 - N2: N2-free iPS-SF1
iPS-SF1 - bFGF: bFGF-free iPS-SF1

Thus it can be seen that, the effects of bFGF on iPS reprogramming efficiency are even higher than those of LIF known in the art.

Example 6: Inhibitory effects of serum on reprogramming of iPS cells cultured in iPS-SF1 medium

As shown in Fig. 5, when 2%, 4%, 6%, 8%, and 10% serums were added into iPS-SF1 medium respectively, serum inhibited the efficiency of iPS reprogramming by 96% as a maximum, which is different from the known prior art.

### Example 7: Effects of other tyrosine kinases on the medium of the present invention

In order to verify whether other tyrosine kinases and estrogens can play a role in inducing reprogramming of iPS cells in the medium of the present invention, after MEF cells were infected with the four-factor viruses, the following media were used instead, wherein the blank control was iPS-SF1 without bFGF, the experimental group was the blank medium (i.e., iPS-SF1 without bFGF) supplemented with various receptor tyrosine kinases (including basic fibroblast growth factor bFGF, epidermal growth factor EGF, vascular endothelial growth factor VEGF, insulin-like growth factor 2 IGF2, and estradiol), and a serum medium (FBS) was used as the negative control of this experiment.

As shown in Fig. 8, except for bFGF, all other tyrosine kinases such as EGF, IGF2, VEGF, and estrogen had comparable effects on the induction of the iPS reprogramming process.

### Example 8: Screening compounds in the iPS process in iPS-SF1 medium

20,000 MEF cells were plated in each well of a twelve-well plate. The medium was iPS-SF1. Then, the cells were infected with viruses as described above in order to be transfected with the four factors. The compounds as shown in Fig. 6 were added into the medium at the following concentrations respectively : PD0325901 (1 µM), CHIR99021 (3 µM), SU5402 (2 µM, EMDbiosciences), Y-27632 (10 µM), vitamin E (25 µM, Sigma), vitamin A (1 µM, Sigma), A83-01 (0.5 µM, EMD), SB203580 (2 µM), Dorspmorphin (3 µM, Sigma), PFF-α (10 µM), TSA (20 nM, Sigma), VPA (1 mM, EMD), JAK inhibitor (0.3 µM, EMD), 5aza-DC (1 µM, Sigma), BIX01294 (1 µM), Bayk8644 (2 µM, EMD).

At day 7 after infection, cells were collected for FACS analysis as described above, and the relative GFP efficiency was measured. It can be seen that, TSA and VPA could significantly enhance the efficiency of iPS reprogramming (100%). However, under conditions using the medium of the present invention, the compounds that had been previously reported as favorable to self-renewal of mouse embryonic stem cells, such as PD0325901, CHIR99021, and SU5402 reduced the efficiency of iPS reprogramming.

## Claims

1. A serum-free medium for inducing pluripotent stem cells, comprising a basal medium, a serum replacement additive formulated by KnockOut Serum Replacement (KOSR) and N2-supplement, leukemia inhibitory factor (LIF), and one or more receptor tyrosine kinase growth factors selected from at least one of bFGF, EGF, IGF2, or VEGF, wherein the bFGF has a concentration of 3 ng/mL to 20 ng/mL in the final medium.

2. The medium of claim 1, wherein the basal medium includes Dulbecco's Modified Eagle's Medium (DMEM), Minimal Essential Medium (MEM), Basal Medium Eagle (BME), F-10, F-12, RPMI 1640, Glasgow's Minimal Essential Medium (GMEM), α Minimal Essential Medium (αMEM), Iscove's Modified Dulbecco's Medium, and M199, preferably DMEM.

3. The medium of claim 1, wherein in the final medium the KOSR has a concentration of about 10%, and the N2-supplement has a concentration of about 0,5%.

4. The medium of any one of claims 1 to 3, wherein the receptor tyrosine kinase growth factors can be substituted by an Estrogen, preferably Estradiol.

5. The medium of claim 1, wherein the bFGF has a concentration of 5ng/mL to 15 ng/mL, most preferred 5ng/mL to 7 ng/mL.

6. The medium of claim 1, wherein in the final medium the EGF has a concentration of about 10 ng/mL.

7. The medium of claim 1, wherein in the final medium the IGF2 has a concentration of about 25 ng/mL.

8. The medium of claim 1, wherein in the final medium the VEGF has a concentration of about 10 ng/mL.

9. The medium of any one of claims 1 to 8, wherein the medium further includes other components suitable for cell growth, wherein the other components are selected from L-glutamine, NEAA MEM, sodium pyruvate, and 2-mercaptoethanol.

10. Use of the medium of any one of claims 1 to 9 in a method for inducing pluripotent stem cells from somatic cells with high effciency, comprising:
(a) introducing one or more stem cell pluripotent factors which are proved to be sufficient to induce pluripotency into somatic cells;
(b) culturing the resulting somatic cells of (a) in the medium of any one of claims 1 to 9 under conditions suitable for cell growth so as to induce the somatic cells into pluripotent stem cells;
(c) detecting and analyzing the induced cells for pluripotency;
(d) picking up pluripotent monoclones of the induced pluripotent stem cells;
(e) culturing the monoclone cells of (d) in an embryonic stem cell medium under conditions suitable for growth of embryonic stem cells.

11. Use of the medium of any one of claims 1 to 9 in a method for screening compounds, comprising
(a) introducing one or more stem cell pluripotent factors into somatic cells;
(b) culturing the resulting somatic cells of (a) in the medium of any one of claims 1 to 9 under conditions suitable for cell growth so as to induce the somatic cells into pluripotent stem cells;
(c) detecting and analyzing the induced cells for pluripotency;
(d) picking up pluripotent monoclones of the induced pluripotent stem cells;
(e) culturing the monoclone cells of (d) in an embryonic stem cell medium under conditions suitable for growth of embryonic stem cells;
(f) screening compounds using the induced pluripotent stem cells cultured
in (d); wherein the compounds are screened for their ability to affect the iPS process and induction conditions or their capability of replacing some or all currently known factors required by the iPS induction process.

## Patentansprüche

1. Serumfreies Medium zur Induktion pluripotenter Stammzellen, umfassend ein Basalmedium, ein Serumersatz-Additiv formuliert aus KnockOut Serum Replacement (KOSR) und N2-Supplement, leukämiehemmenden Faktor (LIF) und einen oder mehrere Rezeptortyrosinkinase-Wachstumsfaktoren, ausgewählt aus wenigstens einem von bFGF, EGF, IGF2 oder VEGF, wobei der bFGF im endgültigen Medium eine Konzentration von 3 ng/ml bis 20 ng/ml aufweist.

2. Medium nach Anspruch 1, wobei das Basalmedium Dulbecco's Modified Eagle's Medium (DMEM), Minimal Essential Medium (MEM), Basal Medium Eagle (BME), F-10, F-12, RPMI 1640, Glasgow's Minimal Essential Medium (GMEM), α Minimal Essential Medium (αMEM), Iscove's Modified Dulbecco's Medium und M 199, vorzugsweise DMEM, umfasst.

3. Medium nach Anspruch 1, wobei im endgültigen Medium das KOSR eine Konzentration von ca. 10 % aufweist und das N2-Supplement eine Konzentration von ca. 0,5 % aufweist.

4. Medium nach einem der Ansprüche 1 bis 3, wobei die Rezeptortyrosinkinase-Wachstumsfaktoren durch ein Östrogen, vorzugsweise Östradiol, ersetzt sein können.

5. Medium nach Anspruch 1, wobei der bFGF eine Konzentration von 5 ng/ml bis 15 ng/ml, ganz besonders bevorzugt 5 ng/ml bis 7 ng/ml, aufweist.

6. Medium nach Anspruch 1, wobei der EGF im endgültigen Medium eine Konzentration von ca. 10 ng/ml aufweist.

7. Medium nach Anspruch 1, wobei der IGF2 im endgültigen Medium eine Konzentration von ca. 25 ng/ml aufweist.

8. Medium nach Anspruch 1, wobei der VEGF im endgültigen Medium eine Konzentration von ca. 10 ng/ml aufweist.

9. Medium nach einem der Ansprüche 1 bis 8, wobei das Medium ferner weitere für das Zellwachstum geeignete Bestandteile umfasst, wobei die weiteren Bestandteile ausgewählt sind aus L-Glutamin, NEAA MEM, Natriumpyruvat und 2-Mercaptoethanol.

10. Verwendung des Mediums nach einem der Ansprüche 1 bis 9 in einem Verfahren zur hocheffizienten Induktion pluripotenter Stammzellen aus somatischen Zellen, umfassend:
(a) Einbringen eines oder mehrerer Stammzell-pluripotenter-Faktoren, die zur Induktion von Pluripotenz hinlänglich geeignet sind, in somatische Zellen;
(b) Kultivieren der resultierenden somatischen Zellen von (a) in dem Medium nach einem der Ansprüche 1 bis 9 unter für das Zellwachstum geeigneten Bedingungen zwecks der Induktion pluripotenter Stammzellen aus den somatischen Zellen;
(c) Nachweisen und Analysieren der induzierten Zellen auf Pluripotenz;
(d) Entnehmen pluripotenter Monoklone der induzierten pluripotenten Stammzellen;
(e) Kultivieren der monoklonalen Zellen von (d) in einem Embryonenstammzellmedium unter für das Wachstum embryonaler Stammzellen geeigneten Bedingungen.

11. Verwendung des Mediums nach einem der Ansprüche 1 bis 9 in einem Verfahren zum Screening von Verbindungen, umfassend:
(a) Einbringen eines oder mehrerer Stammzell-pluripotenter-Faktoren in somatische Zellen;
(b) Kultivieren der resultierenden somatischen Zellen von (a) in dem Medium nach einem der Ansprüche 1 bis 9 unter für das Zellwachstum geeigneten Bedingungen zwecks der Induktion pluripotenter Stammzellen aus den somatischen Zellen;
(c) Nachweisen und Analysieren der induzierten Zellen auf Pluripotenz;
(d) Entnehmen pluripotenter Monoklone der induzierten pluripotenten Stammzellen;
(e) Kultivieren der monoklonalen Zellen von (d) in einem Embryonenstammzellmedium unter für das Wachstum embryonaler Stammzellen geeigneten Bedingungen;
(f) Screening von Verbindungen unter Verwendung der in (d) kultivierten induzierten pluripotenten Stammzellen; wobei das Screening der Verbindungen im Hinblick auf ihre Fähigkeit, den iPS-Prozess und die Induktionsbedingungen zu beeinflussen, oder ihre Fähigkeit, einige oder alle derzeit bekannten, für den iPS-Induktionsprozess erforderlichen Faktoren zu ersetzen, erfolgt.

## Revendications

1. Un milieu sans sérum pour l'induction de cellules souches pluripotentes, comprenant un milieu basal, un additif de remplacement de sérum élaboré à l'aide de remplacement de sérum KnockOut (KOSR) et de supplément N2, du facteur d'inhibition de la leucémie (LIF) et d'un ou plusieurs facteurs de croissance de récepteur à activité tyrosine kinase sélectionné parmi au moins un de bFGF, EGF, IGF2 ou VEGF, le bFGF ayant une concentration de 3 ng/mL à 20 ng/mL dans le milieu final.

2. Le milieu de la revendication 1, le milieu de base comprenant le milieu d'Eagle modifié de Dulbecco (DMEM), le milieu essentiel minimum (MEM), le milieu de base eagle (BME), F-10, F-12, RPMI 1640, le milieu essentiel minimum de Glasgow (GMEM), le milieu essentiel minimum α (αMEM), le milieu de Dulbecco modifié d'Iscove et M199, de préférence DMEM.

3. Le milieu de la revendication 1, le KOSR ayant une concentration d'environ 10 % et le supplément N2 ayant une concentration d'environ 0,5 % dans le milieu final.

4. Le milieu de l'une quelconque des revendications 1 à 3, les facteurs de croissance de récepteur à activité tyrosine kinase pouvant être substitués par un oestrogène, de préférence l'estradiol.

5. Le milieu de la revendication 1, le bFGF ayant une concentration de 5 ng/mL à 15 ng/mL, de préférence de 5 ng/mL à 7 ng/mL.

6. Le milieu de la revendication 1, l'EGF ayant une concentration d'environ 10 ng/mL, dans le milieu final.

7. Le milieu de la revendication 1, l'IGF2 ayant une concentration d'environ 25 ng/mL dans le milieu final.

8. Le milieu de la revendication 1, le VEGF ayant une concentration d'environ 10 ng/mL dans le milieu final.

9. Le milieu de l'une quelconque des revendications 1 à 8, le milieu comprenant en outre d'autres composants adaptés à la croissance cellulaire, les autres composants étant sélectionnés parmi la L-glutamine, un milieu essentiel minimum (MEM) d'acides aminés non essentiels (NEAA), le pyruvate de sodium et le 2-mercaptoéthanol.

10. Utilisation du milieu de l'une quelconque des revendications 1 à 9 dans une méthode pour l'induction de cellules souches pluripotentes à partir de cellules somatiques avec une efficacité élevée, comprenant :
(a) introduire un ou plusieurs facteurs pluripotents de cellules souches qui sont avérés suffisants afin d'induire la pluripotence dans des cellules somatiques ;
(b)cultiver les cellules somatiques résultant de (a) dans le milieu de l'une quelconque des revendications 1 à 9, dans des conditions adaptées à la croissance cellulaire, afin d'induire les cellules somatiques en cellules souches pluripotentes ;
(c) détecter et analyser les cellules induites relativement à la pluripotence ; (d)prendre des monoclones pluripotents parmi les cellules souches pluripotentes induites ;
(e) cultiver les cellules monoclones de (d) dans un milieu de cellules souches embryonnaires dans des conditions adaptées à la croissance de cellules souches embryonnaires.

11. Utilisation du milieu de l'une quelconque des revendications 1 à 9 dans une méthode pour le criblage de composés, comprenant
(a) introduire un ou plusieurs facteurs pluripotents de cellules souches dans des cellules somatiques ;
(b) cultiver les cellules somatiques résultant de (a) dans le milieu de l'une quelconque des revendications 1 à 9, dans des conditions adaptées à la croissance cellulaire, afin d'induire les cellules somatiques en cellules souches pluripotentes ;
(c) détecter et analyser les cellules induites relativement à la pluripotence ;
(d) prendre des monoclones pluripotents parmi les cellules souches pluripotentes induites ;
(c) cultiver les cellules monoclones de (d) dans un milieu de cellules souches embryonnaires, dans des conditions adaptées à la croissance de cellules souches embryonnaires ;
(f) procéder au criblage de composés en utilisant les cellules souches pluripotentes induites cultivées au point (d) ; les composés étant criblés relativement à leur aptitude à avoir un impact sur le processus iPS et sur les conditions d'induction, ou relativement à leur aptitude à remplacer certains ou tous les facteurs actuellement connus et requis par le processus d'induction iPS.
